# EUROPÄISCHE PATENTSCHRIFT

(11) **EP 1 752 167 B1**
(45) Veröffentlichungstag und Bekanntmachung des Hinweises auf die Patenterteilung: **17.09.2008**
(21) Anmeldenummer: 06013293.3
(22) Anmeldetag: 28.06.2006
(51) Int. Cl.: A61L 27/18, A61L 27/30, A61L 27/58, A61L 17/04, A61L 17/06, A61L 17/14, C23C 18/08, C23C 16/06, C23C 18/06

(54) **Resorbierbares Implantat mit Titanbeschichtung**
Resorbable implant with titanium coating
Implant résorbable avec un revêtement de titanium

(30) Priorität: 13.07.2005 DE 102005032604
(43) Veröffentlichungstag der Anmeldung: 14.02.2007
(73) Patentinhaber: GfE Medizintechnik GmbH, 90431 Nürnberg (DE)
(72) Erfinder: Zimmermann, Hanngörg, 91327 Gössweinstein (DE); Heinlein, Markus, Dr., 91327 Gössweinstein (DE)
(74) Vertreter: Hübner, Gerd

(56) Entgegenhaltungen:
- EP-A- 0 603 976
- EP-A- 1 504 773
- EP-A1- 0 897 997
- WO-A-01/12246
- WO-A-20/06017396
- DE-A1- 10 026 540
- DE-A1- 10 121 193
- US-A1- 2002 099 449
- US-A1- 2002 155 212
- US-A1- 2005 060 043
- US-B1- 6 395 326

## Beschreibung

Die Erfindung betrifft resorbierbare, in den Körper einsetzbare medizinische Elemente - insbesondere ein resorbierbares Implantat - mit einem Grundkörper aus einem im Körper des Implantatempfängers resorbierbaren Material. Die möglichen Formen dieser medizinischen Elemente decken alle sinnvollen medizinischen Anwendungen, wie Schrauben und Platten für Knochenfixierungen, Nahtmaterial, Wundklammern, Knochenersatzgranulat, Stents, Weichgewebe-Verstärkungsimplantate, wie beispielsweise Herniennetze, Klammern, Anker und Stapler zur Implantatfixierung, Membransysteme, wie sie in der Zahnheilkunde verwendet werden, Röhrchen als sogenannten Nervenschienen oder Trägermaterial für den Bereich Tissue Engeneering ab. Der Einfachheit halber sollen im folgenden diese medizinischen Elemente unter dem Begriff "Implantat" zusammengefasst werden.

Zum Hintergrund der Erfindung ist festzuhalten, dass resorbierbare Kunststoffe in vielen solchen Bereichen der Implantat-Medizin Anwendung finden, wo eine temporäre Stabilisierung von Gewebe jedweder Art, wie Weichgewebe oder Knochen, bzw. eine zeitlich begrenzte Funktion eines Implantates benötigt wird. Als Beispiel ist auf Implantate zur Fixierung einer Knochenfraktur zu verweisen, wo Knochenschienen und Schraubensysteme für die Zeitdauer der Selbstgeneration des Knochens eingesetzt werden. Dabei muss der Knochen - wie bei einer normalen Fraktur üblich - zunächst ruhig gestellt werden, um wieder zusammen zu wachsen. Danach ist die einen Fremdkörper darstellende Fixierung nicht mehr notwendig und herkömmliche Implantate aus einem beständigen Material müssen im Rahmen einer Nachoperation entfernt werden.

Ein solcher zusätzlicher Eingriff ist zu vermeiden, wenn für die Implantate, wie Nägel oder Schienen ein resorbierbares Material verwendet wird, das nach einer gewissen, durch entsprechende Materialauswahl spezifizierbaren Dauer von wenigen Wochen bis mehreren Monaten in die körperverträglichen Bestandteile Wasser und Kohlendioxid zerfällt, wobei letztere problemlos aus dem Körper ausgeschieden werden.

Ein anderes Beispiel ist der Einsatz von resorbierbarem Nahtmaterial, der nahezu in allen Bereichen der Wundmedizin nützlich ist. Ziel ist es dabei, den Wundverschluss so zu gestalten, dass zunächst bei ausreichender Reißfestigkeit des Fadens eine Wundheilung erfolgen kann. Mit zunehmender Stabilität des körpereigenen Gewebes nimmt aufgrund der Resorption die Festigkeit des Nahtmaterials allmählich ab. Nach einer gewissen Zeit ist es letztendlich komplett aufgelöst.

Schließlich ist noch der Bereich des Tissue Engineerings zu erwähnen, wo poröse Stützkörper aus resorbierbaren Polymeren erstellt werden. Diese werden anschließend mit einer Zellkultur, also beispielsweise Knochen- oder Weichgewebezellen geimpft. Innerhalb der porösen Struktur, die eine große Anlagerungsoberfläche für die Zellen zur Verfügung stellt, ist ein schnelles und formgebendes Wachstum der Zellen gewährleistet. Die anfängliche Festigkeit der Struktur und somit die Formgebung wird zunächst durch den resorbierbaren Stützkörper erzielt. Nach einer gewissen Wachstumsphase nimmt deren Eigenbelastbarkeit zu und der Trägerkunststoff des Stützkörpers löst sich wiederum allmählich auf, bis nur noch reines Zellgewebe bzw. Knochenmaterial vorhanden ist.

Problematisch bei den resorbierbaren Implantaten nach dem Stand der Technik ist die Tatsache, dass das Implantat aufgrund seines Materials vom Empfängerkörper als Fremdkörper erkannt wird. Dies führt zwar in der Regel nicht zu Abstoßungsreaktionen, wie sie bei Organimplantaten oder synthetischen Implantaten zu beobachten sind, jedoch kann ein Anlagern und das Wachstum der körpereigenen Zellen im Bereich des Implantates dadurch beeinträchtigt werden.

Aus der EP 0 603 976 A2 ist es am Beispiel eines Künstlichen Hüftgelenks bekannt, ein resorbier-bares Implantat so zu verbessern, dass bei adäquaten Resorptionseigenschaften ein Anlagern und entsprechendes Wachstum körpereigener Zellen im Bereich des Implantates unterstützt werden.

Der Schaft dieses Hüftgelenkes besteht aus einem resorbierbaren Innenteil, der mit einer porösen Hülle aus Metallfasern bedeckt ist, welche ein Einwachstum von Knochen und damit die Resorption des Innenteils ermöglicht. Die Hülle bedeckt nicht alle Seiten des Implantats.

Damit ist auf den zumindest teilweise aus einem im Körper des Implantatempfängers resorbierbaren Material bestehenden Grundkörper eine nicht resorbierbare Beschichtung aus einem biokompatiblen Material aufgebracht, die diesen nur teilweise bedeckt. Damit weist der Grundkörper beschichtungsfreie Zonen zum Resorptionsangriff des Körpers des Implantatempfängers auf.

Durch die Teil-Beschichtung des Implantates wird einerseits im beschichteten Bereich eine Anlagerung und das Wachstum der körpereigenen Zellen unterstützt, wodurch ein schnelleres Einheilen des Implantats und eine Optimierung der Zellanlagerung erreicht werden. Ferner ist durch die Teilbeschichtung die Resorbierbarkeit steuerbar. Auf dem beschichteten Teil des Implantates wird die Resorption nämlich verlangsamt.

Die aus dem Stand der Technik bekannte Teilabdeckung des resorbierbaren Innenteils eines Implantates mittels einer porösen Hülle aus Metallfasern ist nur bedingt für verschiedenste Einsatzzwecke geeignet. Der Erfindung liegt davon ausgehend die Aufgabe zugrunde, alternative, mit Unterbrechungen oder Offenstellen versehene Beschichtungen bereitzustellen, um somit resorbierbare Implantate einem möglichst breiten Anwendungsspektrum zuzuführen.

Diese Aufgabe wird durch erfindungsgemäße medizinische Elemente mit den in den Ansprüchen 1 bis 3 angegebenen, nicht resorbierbaren Beschichtungen gelöst.

Gemäß einem ersten Aspekt der Erfindung sind zur Bildung von beschichtungsfreien Zonen durch Abtragung der Beschichtung entstandene Unterbrechungen vorgesehen.

Eine erste Alternative dazu sieht Teilbeschichtungen auf dem Grundkörper des Implantats vor, deren Dicke im Bereich von 5 bis 700 nm liegt.

Stattdessen kann auch die Beschichtung als dünne Rudimentärbeschichtung ausgelegt sein, die Offenstellen zur Bildung der beschichtungsfreien Zonen aufweist und eine Dicke von etwa 5 nm zeigt.

Bevorzugte Ausführungsformen des resorbierbaren Implantates mit Teil-Beschichtung sind in den Unteransprüchen sowie der nachfolgenden Beschreibung entnehmbar, in der entsprechende Merkmale, Einzelheiten und Vorteile anhand der beigefügten Zeichnungen näher erläutert werden. Es zeigen:
- Fig. 1: einen höchst schematischen Schnitt durch ein Implantat mit einer einseitigen Titan-Beschichtung,
- Fig. 1B: einen vergrößerten Detailschnitt aus Fig. 1A,
- Fig. 2A: einen höchst schematischen Schnitt durch ein Implantat mit einer allseitigen, mit Unterbrechungen versehenen Titan-Beschichtung,
- Fig. 2B: einen vergrößerten Detailschnitt aus Fig. 2A,
- Fig. 3A: einen höchst schematischen Schnitt durch ein Implantat mit einer dünnen Titan-Rudimentärbeschichtung, und
- Fig. 3B: einen vergrößerten Detailschnitt aus Fig. 3A.

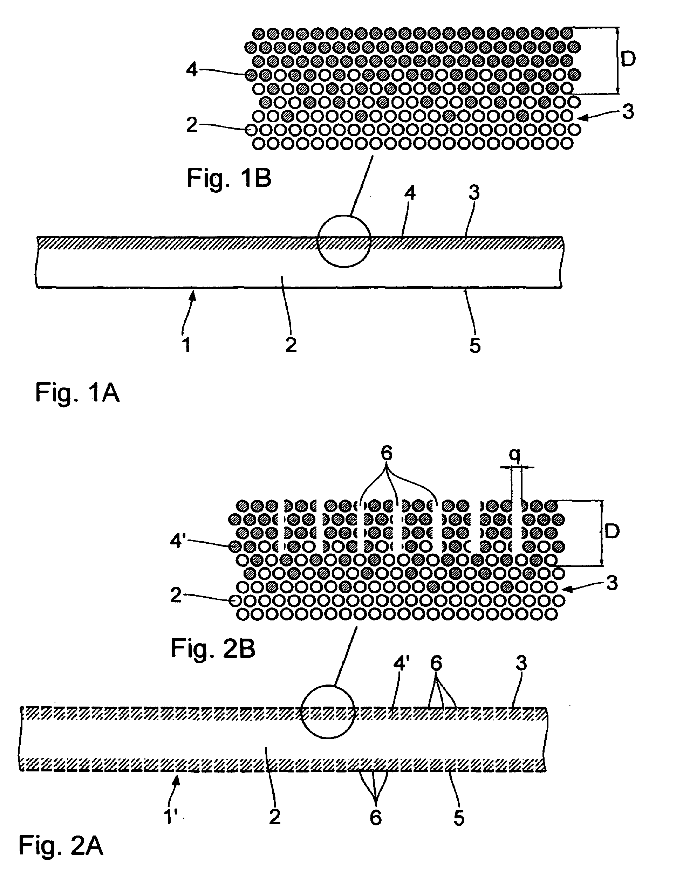

Die Fig. 1A und B zeigen ein Implantat 1, wie es beispielsweise durch eine Knochenschiene realisiert werden kann. Es weist einen Grundkörper 2 auf, der aus einem gängigen resorbierbaren Kunststoffmaterial, wie beispielsweise aus Polylaktiden, deren Copolymeren, Polyglykoliden, deren Copolymeren, Polydioxanonen, Proteinen, wie Casei oder Kollagen, Tricalciumphospaten und dergleichen bestehen kann. Es ist auch denkbar, den Grundkörper nur zumTeil aus einem resorbierbaren Material zu fertigen, beispielsweise bei einem Herniennetz, das je zur Hälfte aus Polypropylen (nicht resorbierbar) und einem resorbierbaren Material besteht.

Die in Fig. 1A und B nach oben weisende Seite 3 des Grundkörpers 2 ist mit einer durchgehenden Beschichtung 4 aus einem titanhaltigen Werkstoff versehen. Diese Seite 3 mit der Beschichtung 4 z. B. eines Knochenschienen-Implantates 1 liegt im implantierten Zustand dem zu stabiliserenden Knochen hingewandt, sodass dort aufgrund der gut bioverträglichen Titanoberfläche das erwähnte Knochenzellenwachstum an den zu stabilisierenden Knochen unterstützt wird.

Auf der der Seite 3 abgewandten Seite 5 der Knochenschiene liegt das bioresorbierbare Material des Grundkörpers 2 völlig offen und ist damit dem Resorptionsangriff aus dem Körper voll ausgesetzt. Dies führt entsprechend zu einem zuverlässigen Abbau des Implantates mit der spezifizierten Zeitkonstante.

Die Dicke D der Beschichtung 4 kann breit zwischen 5 bis 700 nm, vorzugsweise zwischen 10 und 100 nm streuen. Ein praktischer Wert beträgt ca. 30 bis 50 nm. Die Beschichtung selbst wird in einem PACVD-Verfahren aufgebracht, wie es beispielsweise umfassend in der EP 0 897 997 B 1 beschrieben ist. Insoweit bedarf es hier keiner näheren Erörterung des Beschichtungsverfahrens. Als Alternative können andere geeignete Beschichtungsverfahren, wie z. B. ein Sol-Gel-Verfahren, je nach Beschichtungswerkstoff eingesetzt werden.

Bei der in Fig. 2 gezeigten Ausführungsform des Implantates 1' kann es sich beispielsweise um einen Stent oder ein Nervenröhrchen handeln. Bei diesem Implantat 1' ist wiederum ein Grundkörper 2 aus einem oben angegebenen resorbierbaren Material vorgesehen, auf den hier eine allseitige Beschichtung 4' aus einem titanhaltigen Material aufgebracht ist. Durch die allseitige durchbrochene Beschichtung 4' kann die postoperative Zellbesiedlung des Implantates 1' unterstützt werden. Zur Bildung der beschichtungsfreien Zonen wird der Grundkörper 2 an den Stellen maskiert, wo später die Unterbrechungen 6 in der Beschichtung 4' vorhanden sein sollen. Beim Aufbringen der Beschichtung 4' - wiederum beispielsweise mit dem vorgenannten PACVD-Verfahren - werden dann die Unterbrechungen 6 in der Beschichtung 4' ausgespart und durch Entfernen der Maskierung freigelegt. Der offene Querschnitt q der Unterbrechungen kann in der Größenordnung von wenigen Mikrometern bis einigen Millimetern liegen. Die Dicke D der Beschichtung 4' entspricht wiederum etwa 30 bis 50 nm.

Wie anhand der Figuren nicht näher dargelegt ist, können die Unterbrechungen 6 auch nachträglich nach Aufbringen einer durchgehenden Beschichtung 4' durch entsprechendes Abtragen der Beschichtung 4' eingebracht werden.

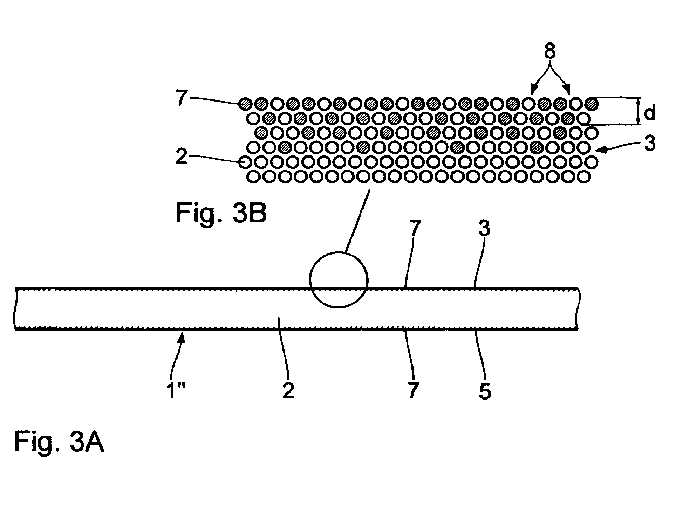

Bei dem in Fig. 3A und B gezeigten Implantat 1'' ist auf dem Grundkörper 2 eine sehr dünne Rudimentärbeschichtung 7 aus einem titanhaltigen Material auf dem Grundkörper 2 aufgebracht. Bei letzerem kann es sich beispielsweise um einen chirurgischen Nahtfaden handeln, bei dem durch die Beschichtung 7 der Wundheilungsprozess beschleunigt wird. Aufgrund der geringen Dicke d der Rudimentärbeschichtung 7 ist diese an der Oberfläche des Grundkörpers 2 nicht durchgehend, sondern porös, wie in Fig. 3B angedeutet ist, sodass die Offenstellen 8 zur Bildung der beschichtungsfreien Zonen am Implantat frei gelassen sind. Über diese Offenstellen kann der Resorptionsangriff aus dem Körper des Implantatempfängers erfolgen.

Der Grundkörper 2 und die Beschichtung 7 des Implantates 1" bestehen wiederum aus den bereits oben angegebenen bioresorbierbaren Werkstoffen bzw. dem PACVD-applizierten titanhaltigen Material.

Bei der dünnen, nicht komplett geschlossenen Rudimentärbeschichtung 7 sind die Offenstellen 8 aufgrund des Beschichtungsauftrages mit einer Dicke von wenigen Atomlagen bis zu den angegebenen 5 nm eher unregelmäßig, wogegen bei der Beschichtung 4' eine regelmäßige Anordnung der Unterbrechungen 6 eher der Fall sein wird. Die Größe der Unterbrechungen 6 bzw. Offenstellen 8 ist nach unten im Wesentlichen durch die jeweils technische Machbarkeit limitiert, wobei für die Maskierung beispielsweise auch lithographische Verfahren denkbar sind.

Die Anwendung der unterschiedlichen Arten von Beschichtungen 4, 4' bzw. 7 ist primär vom jeweiligen Einsatzgebiet des Implantates abhängig. Bei einer Positionierung zwischen unterschiedlichen Grenzflächen z. B. zwischen Weichgewebe und einer Knochenoberfläche, wird eher eine einseitige Beschichtung 4 mit einer gerichteten Orientierung gewählt. Bei Implantaten, die sich innerhalb eines Gewebetyps befinden, wie beispielsweise Nahtmaterial oder sogenannte Trägersysteme, wird die maskierte Beschichtung 4' bzw. Rudimentärbeschichtung 7 auf dem Grundkörper 2 den Vorzug finden. Schließlich kann über das Verhältnis von beschichteter Fläche zu Öffnungsfläche die Resorptionsdauer des Implantates 1, 1', 1" beeinflusst werden. Grundsätzlich sind neben dem erwähnten Titan als Beschichtungsmaterial andere körperverträgliche Metalle, wie Tantal, Hafnium, Zirkonium, Silber oder Niob oder biokompatible Polymere, oder Hydroxylapatit einsetzbar.

## Patentansprüche

1. Resorbierbares, in den Körper einsetzbares medizinisches Element, insbesondere Implantat, umfassend
- einen Grundkörper (2), der zumindest teilweise aus einem im Körper des Implantatempfängers resorbierbaren Material besteht, und
- eine ein biokompatibles Material enthaltende, nicht resorbierbare Beschichtung (4', 7) auf dem Grundkörper (2), die diesen nur teilweise so bedeckt, dass der Grundkörper (2) beschichtungsfreie Zonen zum Resorptionsangriff des Körpers aufweist, wobei die Beschichtung (4') zur Bildung der beschichtungsfreien Zonen mit Unterbrechungen (6) versehen ist,
**dadurch gekennzeichnet, dass** die Unterbrechungen (6) durch eine entsprechende Abtragung der aufgebrachten Beschichtung (4') gebildet sind.

2. Resorbierbares, in den Körper einsetzbares medizinisches Element, insbesondere Implantat, umfassend
- einen Grundkörper (2), der zumindest teilweise aus einem im Körper des Implantatempfängers resorbierbaren Material besteht, und
- eine ein biokompatibles Material enthaltende, nicht resorbierbare Beschichtung (4, 4') auf dem Grundkörper (2), die diesen nur teilweise so bedeckt, dass der Grundkörper (2) beschichtungsfreie Zonen zum Resorptionsangriff des Körpers aufweist, **dadurch gekennzeichnet, dass** die Dicke (D) der Beschichtung (4, 4') im Bereich von 5 bis 700 nm, insbesondere zwischen 10 und 100 nm liegt.

3. Resorbierbares, in den Körper einsetzbares medizinisches Element, insbesondere Implantat, umfassend
- einen Grundkörper (2), der zumindest teilweise aus einem im Körper des Implantatempfängers resorbierbaren Material besteht, und
- eine ein biokompatibles Material enthaltende, nicht resorbierbare Beschichtung (7) auf dem Grundkörper (2), die diesen nur teilweise so bedeckt, dass der Grundkörper (2) beschichtungsfreie Zonen zum Resorptionsangriff des Körpers aufweist, wobei die Beschichtung als dünne Rudimentärbeschichtung (7) ausgelegt ist, die Offenstellen (8) zur Bildung der beschichtungsfreien Zonen aufweist,
**dadurch gekennzeichnet, dass** die Dicke (d) der Rudimentärbeschichtung (7) bei etwa 5 nm liegt.

4. Medizinisches Element nach Anspruch 1, **dadurch gekennzeichnet, dass** die Unterbrechungen (6) einen offenen Querschnitt (q) jeweils im Bereich von wenigen µm bis einigen mm aufweisen.

5. Medizinisches Element nach einem der vorgenannten Ansprüche, **dadurch gekennzeichnet, dass** die Beschichtung ein körperverträgliches Metall, insbesondere Titan, Tantal, Hafnium, Zirkonium, Silber oder Niob oder ein biokompatibles Polymer oder Hydroxylapatit enthält.

6. Medizinisches Element nach Anspruch 5, **dadurch gekennzeichnet, dass** die titanhaltige Beschichtung (4, 4', 8) durch ein PACVD-Verfahren oder ein Sol-Gel-Verfahren aufgebracht ist.

7. Medizinisches Element nach einem der vorgenannten Ansprüche, **dadurch gekennzeichnet, dass** der Grundkörper (2) aus Polylaktiden, deren Copolymeren, Polyglykoliden, deren Copolymeren, Polydioxanonen, Proteinen oder Tricalciumphosphaten besteht.

## Claims

1. Absorbable medical element suitable for insertion into the body, in particular an implant, comprising
- a basic compound (2), which at least partially consists of a material which is absorbable in the body of the implant recipient, and
- a coating (4', 7) on the basic compound (2), wherein said coating (4', 7) contains a biocompatible material, is not absorbable and only covers the basic compound (2) partially in such a manner that the basic compound (2) comprises coating-free zones for an absorption attack by the body, wherein the coating (4') has interruptions (6) in order to form the coating-free zones,
**characterised in that** the interruptions (6) are formed by a corresponding removal of the applied coating (4').

2. Absorbable medical element suitable for insertion into the body, in particular an implant, comprising
- a basic compound (2), which at least partially consists of a material which is absorbable in the body of the implant recipient, and
- a coating (4, 4') on the basic compound (2), wherein said coating (4, 4') contains a biocompatible material, is not absorbable and only covers the basic compound (2) partially in such a manner that the basic compound (2) comprises coating-free zones for an absorption attack by the body,
**characterised in that** the thickness (D) of the coating (4, 4') lies in the range of between 5 and 700 nm, in particular between 10 and 100 nm.

3. Absorbable medical element suitable for insertion into the body, in particular an implant, comprising
- a basic compound (2), which at least partially consists of a material which is absorbable in the body of the implant recipient, and
- a coating (7) on the basic compound (2), wherein said coating (7) contains a biocompatible material, is not absorbable and only covers the basic compound (2) partially in such a manner that the basic compound (2) comprises coating-free zones for an absorption attack by the body, wherein the coating is designed as a thin, rudimentary coating (7) which comprises open sections (8) in order to form the coating-free zones,
**characterised in that** the thickness (d) of the rudimentary coating (7) is approximately 5 nm.

4. Medical element according to claim 1, **characterised in that** the interruptions (6) comprise an open cross-section (q) in each case in the range of between a few µm and several mm.

5. Medical element according to one of the above claims, **characterised in that** the coating contains a body-compatible metal, in particular titanium, tantalum, hafnium, zirconium, silver or niobium, or a biocompatible polymer, or hydroxylapatite.

6. Medical element according to claim 5, **characterised in that** the titanium-containing coating (4, 4', 8) is applied using a PACVD process or a sol-gel process.

7. Medical element according to one of the above claims, **characterised in that** the basic compound (2) consists of polylactides, their copolymers, polyglycolides, their copolymers, polydioxanones, proteins or tricalcium phosphates.

## Revendications

1. Élément médical résorbable destiné à être implanté dans le corps, en particulier un implant, comportant :
- un corps de base (2), qui est réalisé au moins partiellement dans un matériau résorbable dans le corps du receveur de l'implant, et
- un revêtement (4', 7) non résorbable, qui contient un matériau biocompatible et est déposé sur le corps de base (2) et qui couvre celui-ci seulement partiellement, de telle sorte que le corps de base (2) comporte des zones non revêtues pour permettre la résorption par le corps, le revêtement (4') comportant des creux (6) pour former les zones non revêtues,
**caractérisé en ce que** les creux (6) sont formés par un enlèvement correspondant du revêtement (4') déposé.

2. Élément médical résorbable destiné à être implanté dans le corps, en particulier un implant, comportant :
- un corps de base (2), qui est réalisé au moins partiellement dans un matériau résorbable dans le corps du receveur de l'implant, et
- un revêtement (4, 4') non résorbable, qui contient un matériau biocompatible et est déposé sur le corps de base (2) et qui couvre celui-ci seulement partiellement, de telle sorte que le corps de base (2) comporte des zones non revêtues pour permettre la résorption par le corps,
**caractérisé en ce que** l'épaisseur (D) du revêtement (4, 4') se situe dans une plage de 5 à 700 nm, en particulier entre 10 et 100 nm.

3. Élément médical résorbable destiné à être implanté dans le corps, en particulier un implant, comportant :
- un corps de base (2), qui est réalisé au moins partiellement dans un matériau résorbable dans le corps du receveur de l'implant, et
- un revêtement (7) non résorbable, qui contient un matériau biocompatible et est déposé sur le corps de base (2) et qui couvre celui-ci seulement partiellement, de telle sorte que le corps de base (2) comporte des zones non revêtues pour permettre la résorption par le corps, le revêtement étant conçu sous la forme d'un mince revêtement rudimentaire (7), qui comporte des zones dénudées (8) pour former les zones non revêtues,
**caractérisé en ce que** l'épaisseur (d) du revêtement rudimentaire (7) est à peu près de l'ordre de 5 nm.

4. Élément médical selon la revendication 1, **caractérisé en ce que** les creux (6) ont une section (q) ouverte, choisie respectivement dans une plage de quelques µm à quelques mm.

5. Élément médical selon l'une quelconque des revendications précédentes, **caractérisé en ce que** le revêtement contient un métal biocompatible, en particulier le titane, le tantale, le hafnium, le zirconium, l'argent ou le niobium ou un polymère biocompatible ou un hydroxylapatite.

6. Élément médical selon la revendication 5, **caractérisé en ce que** le revêtement (4, 4', 8) contenant du titane est déposé au moyen d'un procédé PACVD ou d'un procédé sol-gel.

7. Élément médical selon l'une quelconque des revendications précédentes, **caractérisé en ce que** le corps de base (2) est réalisé en polylactides, en copolymères de ceux-ci, en polyglycolides, en copolymères de ceux-ci, en polydioxanones, en protéines ou en phosphates de tricalcium.
